# FASCICULE DE BREVET EUROPEEN

(11) **EP 3 217 176 B1**
(45) Date de publication et mention de la délivrance du brevet: **07.08.2019**
(21) Numéro de dépôt: 16000589.8
(22) Date de dépôt: 11.03.2016
(51) Int. Cl.: G01N 33/543, G01N 33/569, G01N 33/68

(54) **PROCEDE IMMUNOENZYMATIQUE PERMETTANT LA DETECTION ET L'IDENTIFICATION SEQUENTIELLES D'ANALYTES**
IMMUNOENZYMATISCHES VERFAHREN, DAS DEN SEQUENZIELLEN NACHWEIS UND DIE SEQUENZIELLE IDENTIFIZIERUNG VON ANALYTEN ERMÖGLICHT
IMMUNOENZYMATIC METHOD ALLOWING THE SEQUENTIAL DETECTION AND IDENTIFICATION OF ANALYTES

(43) Date de publication de la demande: 13.09.2017
(73) Titulaire: Scienion AG, 12489 Berlin (DE)
(72) Inventeur: ZREIN, Maan, 69130 Ecully (FR); GUEYFFIER, Lucie, 69100 Villeurbanne (FR)
(74) Mandataire: v. Bezold & Partner Patentanwälte - PartG mbB

(56) Documents cités:
- WO-A1-02/50537
- WO-A1-93/01307
- US-A- 5 902 727
- US-A1- 2004 241 776
- MAKLER M T ET AL: "APPLICATION OF ELISA AND ELADA ASSAY (DOUBLE SUBSTRATE IMMUNOASSAY)TO RED BLOOD CELL ANTIGENS", TRANSFUSION, AMERICAN ASSOCIATION OF BLOOD BANKS, BETHESDA, MD, US, vol. 21, no. 3, 1 janvier 1981 (1981-01-01), pages 303-312, XP000985163, ISSN: 0041-1132, DOI: 10.1046/J.1537-2995.1981.21381201802.X
- WEI WEI ET AL: "Multianalyte immunoassay chip for detection of tumor markers by chemiluminescent and colorimetric methods", ANALYTICAL AND BIOANALYTICAL CHEMISTRY, SPRINGER, BERLIN, DE, vol. 401, no. 10, 18 septembre 2011 (2011-09-18), pages 3269-3274, XP019978247, ISSN: 1618-2650, DOI: 10.1007/S00216-011-5400-X
- Stanley Lemon ET AL: "Global Infectious Disease Surveillance and Detection: Assessing the Challenges -- Finding Solutions", 2007, The national academies press, XP055290823, pages 171-171, * le document en entier *

## Description

### DOMAINE DE L'INVENTION

La présente invention propose un procédé rapide, fiable et peu coûteux permettant la détection et l'identification d'analytes dans un échantillon.

Elle repose sur la mise en évidence que l'enzyme employée dans un procédé immunoenzymatique de type ELISA est capable de catalyser deux réactions successives, en présence de deux substrats distincts et générer ainsi deux types de signaux distincts.

### ETAT DE LA TECHNIQUE

Les immunoessais permettent de mesurer la présence ou la concentration d'une molécule, appelée « analyte », dans une solution. Ils sont notamment utilisés pour la mesure d'analytes dans des échantillons biologiques à des fins médicales ou de recherche. Ils sont également très utiles dans de nombreuses autres applications utilisant différents types d'échantillons : eaux, extraits de sols ou de plantes pour des applications environnementales et agricoles ; produits de procédés de fabrication (aliments, effluents...) pour des applications industrielles.

Le principe des immunoessais repose sur deux éléments clés :
Le premier est la capacité d'un anticorps à reconnaître et à se fixer à un antigène spécifique, permettant la « capture » de l'analyte visé à partir de sa contrepartie correspondante : si la molécule à détecter est un antigène, le composant utilisé dans l'essai sera un anticorps ; inversement, c'est un antigène qui sera utilisé dans le cas où on cherche à détecter un anticorps.

Le second attribut essentiel d'un immunoessai correspond au moyen de produire un signal qui peut être mesuré en réponse à la fixation de l'anticorps à l'antigène. Ce moyen implique généralement un marqueur détectable, chimiquement lié ou conjugué à des anticorps ou des antigènes utilisés dans le processus de révélation. Selon sa nature, le marqueur est détecté par la radiation, la lumière ou la fluorescence qu'il émet, ou par le changement de couleur qu'il induit au niveau de la solution.

Une des méthodes les plus couramment utilisées pour quantifier des molécules d'intérêt est la détection enzymatique, qui utilise une enzyme comme marqueur. De tels immunoessais sont appelés immunoessais enzymatiques (EIA de l'anglais « *enzyme immunoassays* »). En association avec le marqueur enzymatique, ces essais utilisent des substrats spécifiques, qui sont transformés en un ou plusieurs produits lors de réactions chimiques catalysées par l'enzyme. C'est le résultat de cette transformation qui est mesuré, indiquant si l'analyte est présent dans la solution et, s'il l'est, à quelle concentration.

A titre d'exemple, les immunoessais enzymatiques chromogènes sont basés sur l'utilisation de substrats changeant de couleur lors de la catalyse par l'enzyme. Ainsi, différents types de substrats peuvent être mis en oeuvre :
- soit un substrat soluble lorsqu'une réponse unique est recherchée (absence/présence ou concentration), comme par exemple dans les tests ELISA (de l'anglais « *enzyme-linked immunosorbent assay* ») traditionnels ;
- soit un substrat dit précipitant pour obtenir des réponses multiples permettant la définition de profil à partir de plusieurs paramètres, comme dans les Western Blots.

Le brevet des Etats-Unis 5,902,727 divulgue un procédé de localisation et de quantification d'une substance cible dans un échantillon biologique au moyen d'une sonde liée à une enzyme. La sonde se lie à la substance de cible et génère une substance chromogène ou fluorogène déposable qui détecte la position et une substance chromogène ou fluorogène soluble qui permet une quantification dans le milieu baignant l'échantillon.

Wei Wei et al. (Analytical and Bioanalytical Chemistry, vol. 401, n° 10 (2011), 3269-3274) divulguent une puce pour l'immunoessai d'analytes multiples permettant de détecter des marqueurs tumoraux par des méthodes chimiluminescentes et colorimétriques.

Actuellement et à titre d'exemple, la recherche d'infections dans un échantillon sanguin destiné à la transfusion nécessite de réaliser autant de tests ELISA que d'agents infectieux recherchés, chacun de ces tests ELISA mettant en oeuvre un cocktail d'antigènes et/ou d'anticorps spécifiques de l'infection testée. Une telle méthode est consommatrice de temps, de réactifs mais aussi d'échantillon biologique. Par ailleurs, ce diagnostic se doit par la suite d'être confirmé par une technique plus robuste de type Western Blot.

Il existe donc un besoin évident de développer un nouveau test multifonctionnel et multiparamétrique plus avantageux, notamment dans le domaine du diagnostic.

### DESCRIPTION DE L'INVENTION

Selon un premier aspect, la présente invention vise un procédé selon la revendication 1 de détection et d'identification, dans un échantillon, d'analyte(s) parmi au moins deux analytes, comprenant de manière générale :
- la réalisation d'un premier test immunoenzymatique donnant une réponse positive lorsqu'au moins un analyte est présent dans l'échantillon ;
- la réalisation d'un second test immunoenzymatique mettant en oeuvre le complexe immunoenzymatique formé à la première étape et permettant d'identifier le(s) analyte(s) dans l'échantillon.

Comme il ressort, le procédé selon l'invention comprend deux étapes séquentielles mettant en oeuvre le même échantillon et la même enzyme.

Dans le cadre de l'invention, on entend par échantillon, une composition de nature au moins partiellement indéfinie. En effet, le but du procédé selon l'invention est de déterminer la présence et la nature des analytes de cet échantillon.

L'échantillon en tant que tel peut se trouver naturellement à l'état liquide, ou être mis en solution dans un solvant approprié tel que de l'eau ou un tampon.

En pratique, l'échantillon peut être un fluide biologique, notamment d'origine humaine ou animale. Il peut notamment s'agir de tissus et cellules issus du corps humain ou animal et leurs dérivés, des organes, du sang, ses composants et ses produits dérivés. Il s'agit par exemple de sérum, d'urine, de salive...

Les applications peuvent être à des fins médicales ou de recherche, notamment en rapport avec le diagnostic clinique d'une maladie, le suivi de l'efficacité d'un traitement ou la détection de produits dopants.

Les produits sanguins (sang, sérum, ...) constituent un échantillon d'intérêt particulier.

Dans ce cadre, une application privilégiée est la qualification biologique des dons de sang en termes infectieux, liée aussi bien aux virus qu'aux bactéries. En effet, plusieurs infections transmissibles par la transfusion sanguine sont testées de manière systématique pour assurer la sécurité transfusionnelle. De manière avantageuse, il est recherché au moins l'un des agents suivants :
- Virus de l'immunodéficience humaine (VIH) ;
- Virus de l'hépatite B (VHB) ;
- Virus de l'hépatite C (VHC) ;
- Bactérie tréponème pâle (TP), responsable de la syphilis ;
- Virus T-lymphotropique humain (HTLV).

Toutefois, le procédé selon l'invention peut-être mis en oeuvre sur d'autres types d'échantillons tels que :
- des échantillons naturels : eaux, extraits de sols ou de végétaux (plantes), notamment pour des applications environnementales et agricoles ;
- des produits de procédés de fabrication : aliments, effluents..., notamment pour des applications industrielles. Dans ce cadre, il peut permettre entre autre de détecter la présence de contaminants dans des échantillons alimentaires ou environnementaux.

Dans le cadre de l'invention, la notion d'analyte s'entend comme un composé ou une molécule à analyser, au niveau de sa présence (test qualitatif) ou de sa concentration (test semi-quantitatif ou quantitatif).

Dans le cadre de l'invention, les tests mis en oeuvre reposent sur la reconnaissance entre un analyte que l'on cherche à détecter ou à identifier et une molécule partenaire, appelée partenaire de liaison, avec lequel s'établit une interaction moléculaire spécifique.

En pratique, le partenaire de liaison de l'analyte d'intérêt est adsorbé (coaté) à la surface d'un support et la mise en contact de l'échantillon, s'il contient l'analyte, entraîne son interaction spécifique avec le partenaire, formant ainsi un complexe immobilisé à la surface du support, y compris après élimination de l'échantillon et éventuel lavage du support qui a pour objectif l'élimination des excédents non liés.

L'un des partenaires du complexe ainsi formé est un anticorps. On parle alors de test immunologique ou d'immunoessai.

Dans le cadre de l'invention, il peut aussi bien s'agir d'un anticorps monoclonal ou polyclonal, d'origine humaine ou animale, ou de fragments d'anticorps.

L'autre partenaire du complexe peut être un antigène ou toute autre molécule susceptible d'induire la production d'anticorps. De telles molécules sont par exemple des haptènes, à savoir des molécules de faible poids moléculaire antigéniques, c'est-à-dire susceptibles d'être reconnues par le système immunitaire mais non immunogènes, sauf si elles sont couplées à une molécule porteuse dite « *carrier* ». Il peut par exemple s'agir de molécules chimiques (vitamines, hormones, pesticides...) ou d'acides aminés.

Un antigène peut être une protéine native ou recombinante, ou un fragment de protéine présentant au moins un épitope.

A titre d'illustration et en rapport avec un complexe anticorps/antigène, différents cas de figure peuvent se présenter :
- les analytes recherchés sont des anticorps ; les partenaires de liaison sont des antigènes ;
- les analytes recherchés sont des antigènes ; les partenaires de liaison sont des anticorps ;
- les analytes recherchés sont un mélange d'anticorps et d'antigènes ; les partenaires de liaison sont les antigènes et les anticorps correspondants.

Dans le cas particulier de l'analyse des échantillons de sang pour la transfusion, des couples antigènes/anticorps susceptibles d'être utilisés pour « signer » des infections d'intérêt sont les suivants :

**Tableau 1 : Combinaison d'anticorpsantigènes pour la détection d'agents infectieux dans les produits sanguins**

| Agent infectieux | Paramètres à détecter (analyte) | Marqueurs pour la détection (partenaire d'analyte) |
|---|---|---|
| VIH (virus de l'immunodéficience humaine) | Anticorps : | Antigènes dérivés de : |
| | *2 anti-protéines de l'enveloppe | *gp120, gp105, gp36, gp41 |
| Types 1 & 2 | *1 anti-protéine gag | *p24, p17 |
| Sous-type O | *1 anti-protéine pol | *p31 |
| | Antigène p24 | Anticorps anti-p24 |
| VHC (virus de l'hépatite C) | Anticorps anti-protéines : | Antigènes dérivés de : |
| | *structurelles | *Core, E2 |
| | *non-structurelles | *NS3, NS4, NS5 |
| VHB (virus de l'hépatite B) | Anticorps anti-protéine core | Antigène dérivé de HBc |
| | Antigène de surface | Anticorps anti-HBsAg |
| TP (bactérie tréponème pâle, responsable de la syphilis) | Anticorps : | Antigènes dérivés de : |
| | *anti-lipoprotéines membranaires | *TpN47, TpN17, TpN15 |
| | *anti-protéine membranaire | *TmpA |
| HTLV (virus T-lymphotropique humain) | Anticorps : | Antigènes dérivés de : |
| | *anti-protéines GAG | *p19, p24 |
| Types 1 & 2 | *anti-protéines de l'enveloppe | *gp46, gp21 |

Les partenaires de liaison utilisés pour la détection des analytes d'intérêt sont donc immobilisés sur un support. Un tel support est par définition une surface solide et peut prendre différentes formes : puits de microplaques, lame de microscope, microsphères, bandelettes, ... Selon un mode de réalisation particulier, il s'agit d'un puits, voire d'une série de puits, telle que celle des microplaques, qui permettent de réaliser un grand nombre de tests en parallèle et d'obtenir un résultat colorimétrique en une seule lecture.

Le matériau utilisé pour le support est tel qu'il permet la fixation du partenaire de liaison de l'analyte. Une telle fixation peut être réalisée par adsorption passive et hydrophobe, ou au contraire par liaison covalente obtenue par traitement de la surface (par exemple fonctionnalisation chimique).

En pratique et comme déjà dit, un tel support peut être une plaque à 96 puits ou davantage (par exemple 384 puits), réalisée en matière plastique, telle que celles commercialisées par exemple par Corning, Greiner ou Dynatech. Selon un mode de réalisation particulier, une solution contenant le partenaire de liaison de l'analyte, éventuellement diluée dans un tampon à une concentration adéquate, est déposée à la surface du support, avantageusement à l'aide d'une pipette, afin de réaliser des dépôts réguliers et reproductibles. Une nuit d'incubation à 4 °C, 25 °C ou à 37 °C suffit alors à assurer la liaison. De manière générale, on parle de support coaté (ou revêtu ou sensibilisé) à l'aide du partenaire de liaison de l'analyte.

Selon l'invention, le support est avantageusement recouvert d'au moins deux composants destinés à interagir spécifiquement avec au moins deux analytes distincts. En pratique, ces analytes peuvent être issus de la même source, par exemple du même agent infectieux, ou de sources distinctes, par exemple de deux agents infectieux. En outre, ces analytes peuvent être de même nature (par exemple deux antigènes ou au contraire deux anticorps) ou de nature distincte.

De manière générale, les solutions des partenaires de liaison des analytes déposées sur le support en au moins deux dépôts distincts appelés spots, permettent une analyse aisée du résultat obtenu à l'issue du procédé selon l'invention.

A titre d'exemple, la surface disponible dans le fond d'un puits d'une plaque à 96 puits est typiquement de l'ordre de 35 mm². A l'aide d'un matériel adapté, il est possible de réaliser des dépôts dont la surface est de l'ordre de 0,1 à 1,0 mm². Ainsi et sur un tel support, il peut être envisagé de faire entre 2 et au moins 100 dépôts distincts.

En termes de volumes, le volume déposé pour réaliser le spot ne doit avantageusement pas excéder 300 µl. En pratique, des dépôts de 1 nl permettent de réaliser une matrice d'au moins 30 dépôts individualisés.

Le nombre et la nature des dépôts peuvent donc varier, ceux-ci devant permettre la détection d'au moins deux analytes distincts. Dans le cas de 3 dépôts par exemple, on peut envisager la détection de 3 analytes distincts ou la détection de deux analytes distincts et la réplication d'un dépôt.

De manière caractéristique, les deux tests immunologiques du procédé selon l'invention mettent en oeuvre une même enzyme pour révéler l'interaction spécifique entre l'analyte contenu dans l'échantillon et son partenaire de liaison. Il s'agit donc de tests immunoenzymatiques. Le principe général bien connu de l'homme du métier est que l'enzyme est conjuguée à un composant capable d'interagir spécifiquement avec l'analyte.

A titre d'exemple, lorsque les analytes recherchés sont des anticorps d'origine humaine, l'enzyme est conjuguée à un anticorps anti-anticorps humain. *A contrario,* lorsque l'analyte recherché est un antigène, l'anticorps spécifique de cet antigène est conjugué à l'enzyme.

Il est à noter qu'en présence d'analytes de nature différente, le conjugué enzymatique peut être constitué d'un mélange de composants, chacun capable d'interagir spécifiquement avec un analyte. La seule condition est que tous les composants soient conjugués à la même enzyme.

Dans le cas où l'analyte est présent dans l'échantillon testé, il y a donc fixation de l'enzyme au niveau du complexe analyte/partenaire de liaison de l'analyte, formant un complexe immunoenzymatique détectable grâce à son activité et au type de réaction qu'elle catalyse :
- radiochimique, provoquant de la radioactivité ;
- fluorophore, émettant un signal fluorescent ;
- luminescent, générant de la lumière ;
- chromogène, induisant un changement de couleur.

De manière préférentielle, l'enzyme mise en oeuvre dans le cadre de la présente invention dispose d'au moins deux substrats différents, générant des signaux différents.

Selon la présente invention, l'enzyme est une enzyme utilisant des substrats chromogènes, choisie avantageusement dans le groupe suivant : peroxydase de raifort (HRP, de l'anglais *« horseradish peroxidase* »), phosphatase alcaline (AP, de l'anglais « *alkaline phosphatase* »), glucose oxydase, uréase, phosphatase acide, galactosidase acétylcholinestérase, malate déshydrogénase, glucose-6-phosphate déshydrogénase.

De manière connue, ces enzymes sont capables de réagir aussi bien avec des substrats solubles ou insolubles (dits précipitants). Dans le cas d'un substrat soluble, le changement de couleur est homogène dans la solution, alors que pour un substrat précipitant, le changement de couleur est localisé au site de formation de l'agrégat.

Dans le cas d'une utilisation de substrats chromogènes, un changement de couleur peut être détecté à l'oeil nu, par analyse d'images ou par la mesure de la densité optique (DO) à l'aide d'un spectrophotomètre.

Le substrat connu de la peroxydase de raifort est le peroxyde d'hydrogène (H₂O₂). L'enzyme hydrolyse cette molécule pour libérer un oxygène réactif servant à oxyder d'autres molécules telles que la tétraméthylbenzidine (TMB) (soluble dans le DMSO, ou diméthylsulfoxyde) et la tétraméthylbenzidine ou TMB (insoluble), orthophénylène diamine. Ces molécules, incolores au départ deviennent colorées suite à leur oxydation.

Parmi les substrats connus de la phosphatase alcaline, on peut citer le p-nitrophénylphosphate (PNPP) (soluble) et le 5-bromo-4-chloro-3-indolylphosphate/chlorure de nitrobleu de tétrazolium (BCIP/NBT) (insoluble).

De manière privilégiée, les deux substrats mis en oeuvre dans les deux étapes du procédé selon l'invention sont de nature différente et génèrent des signaux distincts.

De manière adaptée, le premier substrat génère un signal global de type binaire (positif/négatif), alors que le second substrat génère un signal différencié et localisé.

Avantageusement, le procédé selon l'invention combine un substrat soluble et un substrat précipitant.
De manière plus spécifique, la première étape du procédé selon l'invention met en oeuvre un substrat chromogène soluble et la seconde étape un substrat chromogène précipitant.

Selon le procédé de l'invention, la première étape consiste donc en un criblage : il s'agit de détecter la présence d'au moins l'un des analytes recherchés grâce à la mise en oeuvre d'un premier test immunoenzymatique donnant une réponse positive lorsqu'au moins un analyte est présent dans l'échantillon.

En pratique, la réaction de l'enzyme avec le substrat en présence de l'analyte se caractérise par l'émission d'un signal, ledit signal signant la liaison de l'analyte et donc sa présence dans l'échantillon assimilée à une réponse positive.

Selon un mode de réalisation particulier, cette première étape correspond à un test immunologique, plus précisément immunoenzymatique par exemple de type ELISA, comprenant les étapes suivantes :
a) la mise en contact d'un échantillon avec un support coaté à l'aide des partenaires de liaison des analytes recherchés ;
b) l'élimination de l'échantillon ;
c) l'ajout de(s) conjugué(s) enzymatique(s) ;
d) l'ajout d'un premier substrat de l'enzyme.

De manière inattendue, il a été constaté qu'un second test immunoenzymatique pouvait être réalisé directement, par simplement ajout d'un autre substrat de l'enzyme. Ainsi, le procédé selon l'invention comprend avantageusement une étape supplémentaire (étape e) consistant à ajouter un second substrat de l'enzyme en présence. Ce second substrat, lors de la catalyse générée par l'enzyme, émet un second signal différent du signal émis par la catalyse du premier substrat. Les premier et second tests immunoenzymatiques, bien que mis en oeuvre à l'aide de la même enzyme et du même échantillon, génèrent donc des réponses différentielles et des informations complémentaires.

Il ressort que de manière avantageuse, aussi bien l'échantillon que les réactifs n'ont pas à être remplacés.

L'étape a) correspond à la mise en contact de l'échantillon, avantageusement sous forme d'une solution, avec un support coaté comme détaillé ci-dessus. Typiquement, l'échantillon est déposé à l'aide d'une pipette, éventuellement de manière automatisée. Le volume déposé est avantageusement contrôlé, assurant ainsi un caractère quantitatif ou du moins semi-quantitatif au procédé selon l'invention.

L'étape b) a pour but d'éliminer la phase liquide en présence mais également toutes les molécules de l'échantillon qui n'ont pas interagi avec les molécules coatées sur le support, à savoir les partenaires de liaison des analytes recherchés. En pratique, il est attendu que seuls les analytes recherchés, présents dans l'échantillon, soient retenus sur le support via la formation d'un complexe immunologique.

Le ou les conjugués enzymatiques sont ajoutés à l'étape c) du procédé selon l'invention. L'enzyme mise en oeuvre est conjuguée à des composants susceptibles de se lier aux analytes recherchés. Dans le cas de la formation d'un complexe immunologique à l'étape b), un complexe immunoenzymatique est formé à l'issue de l'étape c).

Selon un mode de réalisation particulier, entre les deux tests immunoenzymatiques, le support est lavé à l'aide d'une solution de lavage, par exemple de l'eau ou un tampon, permettant d'éliminer le substrat mis en oeuvre dans le premier test, sans affecter le complexe immunoenzymatique formé à la première étape (partenaire de liaison de l'analyte / analyte / enzyme conjuguée).

Plus précisément et selon un mode de réalisation particulier, un lavage est réalisé entre les étapes suivantes telles que définies ci-dessus en rapport avec le procédé selon l'invention :
- entre l'étape b) et c) et/ou ;
- entre l'étape c) et d) et/ou ;
- entre l'étape d) et e).
Comme déjà dit, le lavage entre les étapes d) et e), c'est-à-dire entre les deux tests immunoenzymatiques, apparaît comme particulièrement critique dans la mesure où il correspond au changement de substrat et assure donc la fiabilité des réponses obtenues.

Selon un mode de réalisation particulier, le résultat obtenu à l'issue du premier test immunoenzymatique est de type binaire :
- il donne une réponse positive lorsqu'au moins un analyte est présent dans l'échantillon ;
- il donne une réponse négative lorsqu'aucun des analytes recherchés n'est présent dans l'échantillon.
Cette première étape est donc assimilée à un procédé de détection de la présence d'analytes dans un échantillon.

A titre d'illustration et dans le cas où deux analytes sont recherchés, la réponse obtenue à l'issue de la première étape d'un procédé selon l'invention est positive dans le cas où l'échantillon contient un ou deux de ces analytes et négative s'il n'en contient aucun.

A l'issue de ce premier test enzymatique et notamment dans le cas d'une réponse positive, l'information n'est donc que partielle : il reste à déterminer si l'échantillon contient un ou deux analytes et lesquels. Le second test immunoenzymatique est donc assimilé à un procédé d'identification du ou des analytes présents dans un échantillon.

Comme déjà dit, le substrat mis en oeuvre dans cette première étape est un substrat chromogène soluble. Ainsi, le résultat obtenu à l'issue de cette étape est de type binaire : en l'absence de changement de couleur du milieu réactionnel (liquide), il est conclu qu'aucun des analytes n'est présent dans l'échantillon, alors qu'un changement de couleur signe la présence d'au moins un analyte dans l'échantillon.

De manière complémentaire au substrat chromogène soluble, le substrat mis en oeuvre dans le second test est un substrat précipitant. A l'issue de cette étape et par localisation de la couleur, il est donc possible de savoir quel analyte est présent dans l'échantillon.

Le second test immunoenzymatique du procédé selon l'invention peut être mis en oeuvre de manière systématique et ainsi confirmer les résultats obtenus à l'issue du premier test immunoenzymatique.

Selon un mode de réalisation particulier, le second test immunoenzymatique n'est réalisé que lorsque la réponse du premier test immunoenzymatique est positive, c'est-à-dire qu'au moins un analyte est détecté. En pratique, l'étape e) du procédé tel que décrit ci-dessus, à savoir l'ajout d'un second substrat de l'enzyme en présence, n'est mise en oeuvre que dans ce cas de figure. De manière incontestable, ceci permet d'économiser des réactifs et du temps.

En d'autres termes, ce second test immunoenzymatique est effectué sur les échantillons identifiés comme positifs dans le premier test. Il peut bien sûr s'agir de faux positifs qui sont alors discriminés dans le cadre de ce second test.

Il ressort clairement que le procédé selon l'invention, simple et peu coûteux, prend tout son sens lorsqu'il est mis en oeuvre en parallèle sur différents échantillons susceptibles de contenir au moins un des analytes recherchés. Selon un mode de réalisation particulier, un moins un de ces échantillons contient au moins un des analytes testés. Avantageusement, le second test immunoenzymatique n'est mis en oeuvre que sur ces échantillons.

Selon un autre aspect, la présente invention vise un kit ou une trousse pour la mise en oeuvre du procédé selon l'invention. De manière adaptée, ce kit ou trousse comprend :
- un support sur lequel sont coatés les partenaires de liaison d'au moins deux analytes ;
- une enzyme conjuguée à une ou plusieurs molécules aptes à interagir spécifiquement avec lesdits analytes ;
- au moins deux substrats de ladite enzyme, un soluble et un précipitant.

Le support, les partenaires de liaison des analytes, l'enzyme conjuguée et les substrats présentent avantageusement les caractéristiques détaillées ci-dessus.

Un lavage étant avantageusement réalisé à plusieurs étapes du procédé selon l'invention, la trousse peut également contenir une solution de lavage. Ladite solution de lavage est avantageusement adaptée à l'enzyme de la trousse, c'est-à-dire non susceptible d'affecter son activité. Il s'agit généralement d'une solution tampon.

De manière avantageuse, le support est avantageusement un puits d'une microplaque à puits multiples (96 ou 384 puits), offrant la possibilité de tester de nombreux échantillons en parallèle.

Il ressort de ce qui précède que le procédé et la trousse selon l'invention sont particulièrement utiles pour l'analyse de plusieurs sources d'analytes dans un échantillon ou pour l'analyse simultanée de plusieurs échantillons.

Comme déjà dit, une application privilégiée du procédé et de la trousse selon l'invention concerne l'analyse des produits sanguins dans le cadre de la sécurité transfusionnelle, notamment du point de vue infectieux.

De nombreuses autres applications peuvent être envisagées, notamment :
- la recherche exploratoire de plusieurs infections en cas de suspicion d'infection sans symptômes suffisamment précis ;
- la recherche d'anticorps irréguliers dirigés contre des hématies de différents groupes sanguins ;
- la recherche d'un ensemble de paramètres inflammatoires pour confirmer l'état inflammatoire d'un patient, orienter le diagnostic de la cause de l'inflammation et suivre son évolution ;
- la recherche d'un ensemble de marqueurs de l'auto-immunité afin d'établir un diagnostic d'une maladie auto-immune et choisir un traitement adapté ;
- l'investigation multi-allergènes chez des patients présentant des réactions allergiques ;
- la recherche d'anticorps irréguliers dirigés contre les antigènes érythrocytaires chez les femmes enceintes rhésus négatif et dans les dons de sang ;
- la recherche d'anticorps spécifiques (anti-CMV, anti-tétanos...) dans des dons de sang pour utilisation à des fins thérapeutiques ;
- le dosage d'autres types de protéines dans les dons de sang (ferritine, transaminases...) pour détecter des pathologies asymptomatiques pouvant avoir des impacts négatifs chez des receveurs de produits sanguins.

Le procédé selon l'invention offre un test multiparamétrique et multifonctionnel qui présente de nombreux avantages : il fait le lien opérationnel entre des besoins diagnostics portant sur des volumes importants à traiter dans un délai court et des solutions multiplexes. Il met à profit les différents atouts de l'ELISA tout en lui adjoignant les avantages du multiplex, en particulier :
- le traitement à haut-débit en phase de dépistage (test 1) ;
- la capacité multiplexe offerte par un traitement unique pour un résultat multi-paramètres (test 2) ;
- la double fonctionnalité qui permet de réaliser, immédiatement après la phase de dépistage et au sein du même flux d'opérations, une étape de confirmation dans les seuls cas où cela est nécessaire.

La présente invention va être illustrée plus avant à l'aide des exemples de réalisation qui suivent, à l'appui des figures annexées. Toutefois, ceux-ci n'ont aucune portée limitative.

### LEGENDES DES FIGURES :

La figure 1 est une représentation schématique du fond d'un puits dans lequel 30 micro-spots ont été déposés. Les numéros correspondent aux antigènes tels que définis dans le tableau 1 ci-dessous.
La figure 2 illustre l'image du fond du puit à la phase de précipitation (en haut) et le dessin des spots allumés (en bas) après analyse, à l'aide du procédé selon l'invention, de 3 échantillons infectés (A/ C+ ; B/ IB371 ; C/ IB342).

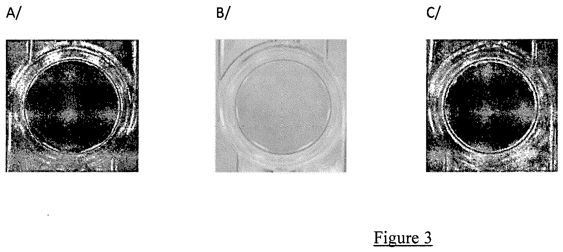

La figure 3 illustre l'image du fond du puit à la phase de précipitation après analyse, à l'aide du procédé selon l'invention, de 3 échantillons non infectés (A/ C- ; B/ IB802 ; C/ IB847).

### EXEMPLES DE REALISATION

### I/ Matériel et Méthodes :

### a) Préparation des microplaques

Chacun des réactifs sélectionnés a été déposé sur 3 micro-emplacements (micro-spots) de chacun des 96 puits d'une microplaque ELISA. Une même expérience est donc réalisée en triplicat.

Pour chaque micro-spot, une quantité de 1 nl à une concentration de 0,5 mg/ml dans un tampon de marquage phosphaté de pH 7,4 a été déposée.

Les microplaques ont été séchées à 37 °C pendant la nuit, puis ont été incubées dans un tampon de saturation d'hydrolysat de caséine à température ambiante pendant une heure, avant d'être lavées avec un tampon de lavage (tampon phosphate salin/Tween, PSB-T) .

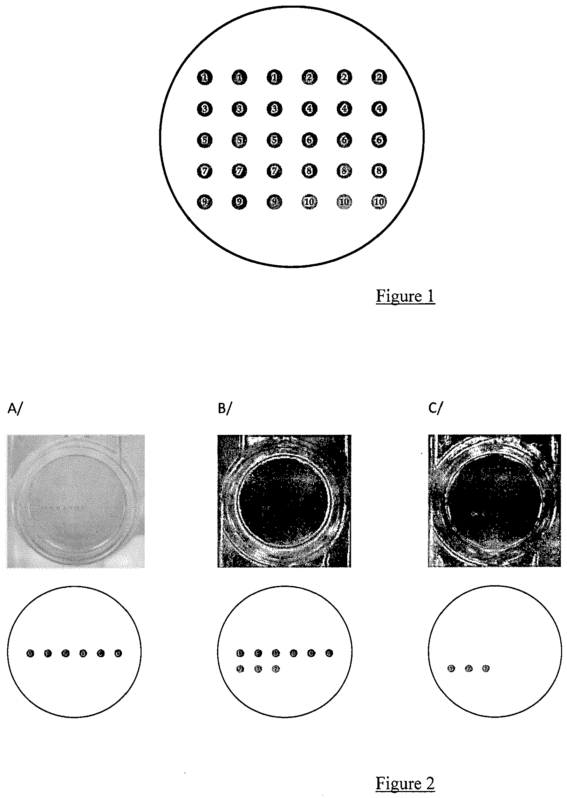

La figure 1 représente le fond d'un puits ainsi préparé, avec une matrice des 30 micro-spots répartis comme suit :

**Tableau 2 : Liste des antigènes déposés sur le support**

| **NUMERO DE DEPOT DANS LE PUITS** | **NOMBRE DE DEPOTS REALISES** | **AGENT INFECTIEUX RECHERCHE DANS L'ECHANTILLON** | **ANTIGENE SPÉCIFIQUE DE L'INFECTION DEPOSE DANS LE PUITS** |
|---|---|---|---|
| 1 | 3 | Virus de l'hépatite C (VHC) | Peptide couplé à la BSA, dérivé de la protéine core |
| 2 | 3 | VHC | Peptide dérivé de la protéine core |
| 3 | 3 | VHC | Peptide couplé à la BSA, dérivé de la protéine NS4 |
| 4 | 3 | VHC | Peptide dérivé de la protéine NS4 |
| 9 | 3 | VHC | Protéine recombinante dérivée de la protéine NS3 |
| | | | |
| 5 | 3 | Virus X | Peptide couplé à la BSA, dérivé de la protéine de *Trypanosoma cruzi* réagissant avec des anticorps d'un virus inconnu |
| 6 | 3 | Virus X | Peptide biotinylé, dérivé de la protéine de *Trypanosoma cruzi* réagissant avec des anticorps d'un virus inconnu |
| | | | |
| 7 | 3 | Virus de l'immuno-déficience humaine (VIH) | Protéine recombinante dérivée de la protéine P24 (VIH-1) |
| 8 | 3 | VIH | Peptide dérivé de la protéine de l'enveloppe GP36 (VIH-2) |
| | | | |
| 10 | 3 | BSA (contrôle) | Albumine sérique bovine |

Ainsi, chaque numéro représente un antigène spécifique d'une infection donnée. En pratique, un puits permet de détecter en triplicat, dans un échantillon donné, la présence éventuelle de 10 anticorps distincts reconnaissants les 10 antigènes listés dans le tableau 1 ci-dessous, permettant le diagnostic de 3 infections distinctes (VHC, virus X, VIH). L'utilisation d'une plaque à 96 puits permet l'analyse simultanée et de manière automatisée de 96 échantillons (différents ou identiques).

### b) Application des échantillons

Une série d'échantillons a été sélectionnée sur la base de leur statut sérologique :
- soit exempts des infections retenues dans l'expérience,
- soit présentant une ou deux infections (virus X et/ou VIH) dont des antigènes sont présents sur la matrice.
Ces échantillons ainsi sélectionnés ont été testés parallèlement sur l'essai multiplex et sur une série d'essais ELISA monoparamétriques utilisant chacun un des réactifs composant l'essai multiparamétrique, afin de valider les résultats obtenus en multiplex.

Les conditions de test suivantes ont été testées pour l'essai multiparamétrique :
- dilution des échantillons au 1:20 dans un tampon de dilution à pH 7,5 ;
- ajout, dans les puits, de 100 µl d'échantillon dilué ou de contrôles et incubation pendant une heure à température ambiante sous agitation orbitale de 900 rpm ;
- lavage des puits avec un tampon de lavage (PBS-T 3x) pour éliminer les anticorps non fixés.

### c) Conjugué enzymatique

L'enzyme peroxydase de raifort, conjuguée à des anticorps anti-IgG (H+L) humaines a été diluée au 1:6 000 dans un tampon conjugué de pH 7,5. Ensuite, 100 µl de conjugué dilué ont été ajoutés dans les puits, avant incubation d'une heure à température ambiante. Les puits ont ensuite été lavés avec un tampon de lavage (PBS-T 0,05 % 3x).

### d) Etape 1 : Substrat soluble

Un substrat chromogène soluble (diméthylsulphoxide (DMSO)-tétraméthylbenzidine (TMB)) a été dissous dans un tampon substrat de pH 4,3, à une concentration de 0,4 mg/ml. 100 µl de cette solution ont été ajoutés au puits ainsi que du peroxyde d'hydrogène. Les puits ont été incubés pendant 30 minutes à température ambiante à l'abri de la lumière. L'absorbance a été mesurée à 630 nm sous agitation dans un lecteur de microplaques à 96 puits et l'analyse des résultats réalisée par le logiciel d'analyse. Les puits ont ensuite été lavés avec du tampon de lavage (PBS-T 1x).

### e) Etape 2 : Substrat insoluble

Une quantité de 100 µl de substrat chromogène insoluble (tétraméthylbenzidine ou TMB) a été ajoutée dans les puits, avant incubation pendant 20 minutes à température ambiante et à l'abri de la lumière.

### II/ Résultats :

Les résultats suivants ont été obtenus avec l'essai multiplex utilisé en deux étapes comme décrit ci-dessus et avec les essais monoparamétriques :

### II-1 Echantillons infectés :

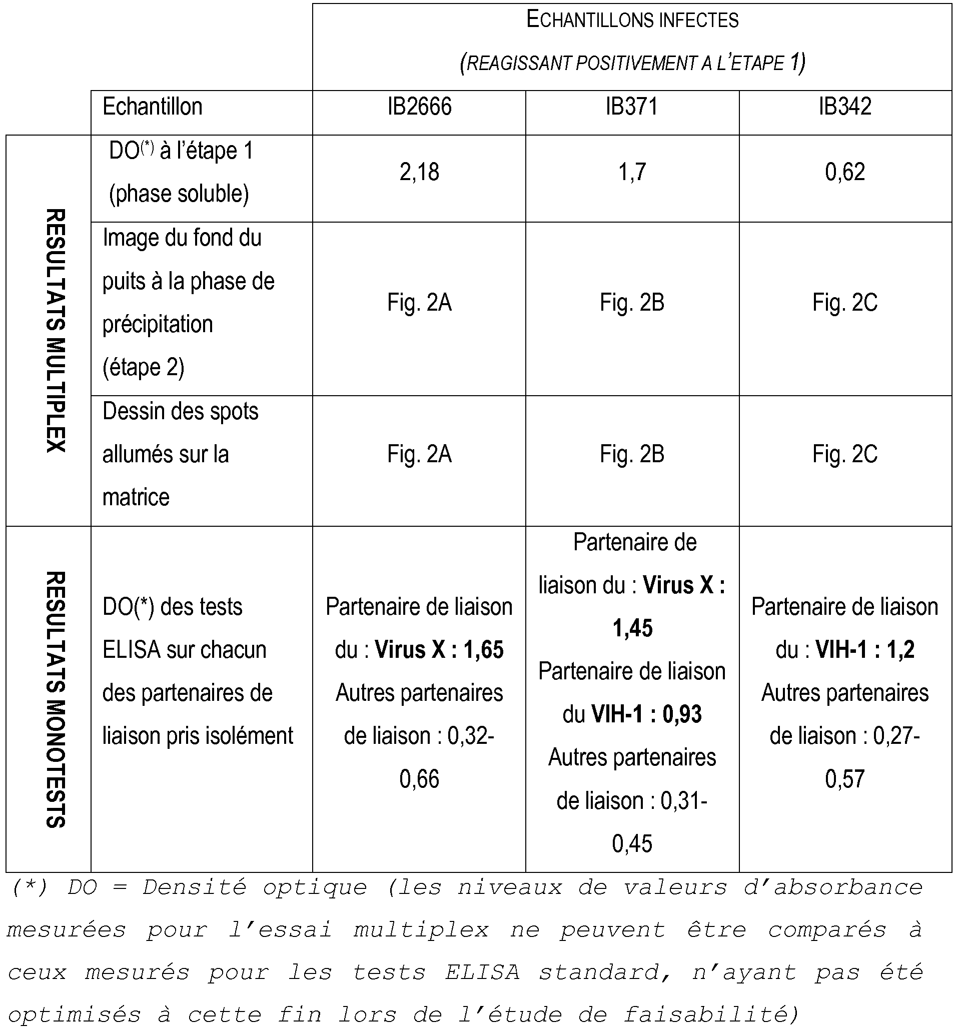

### II-2 Echantillons non infectés :

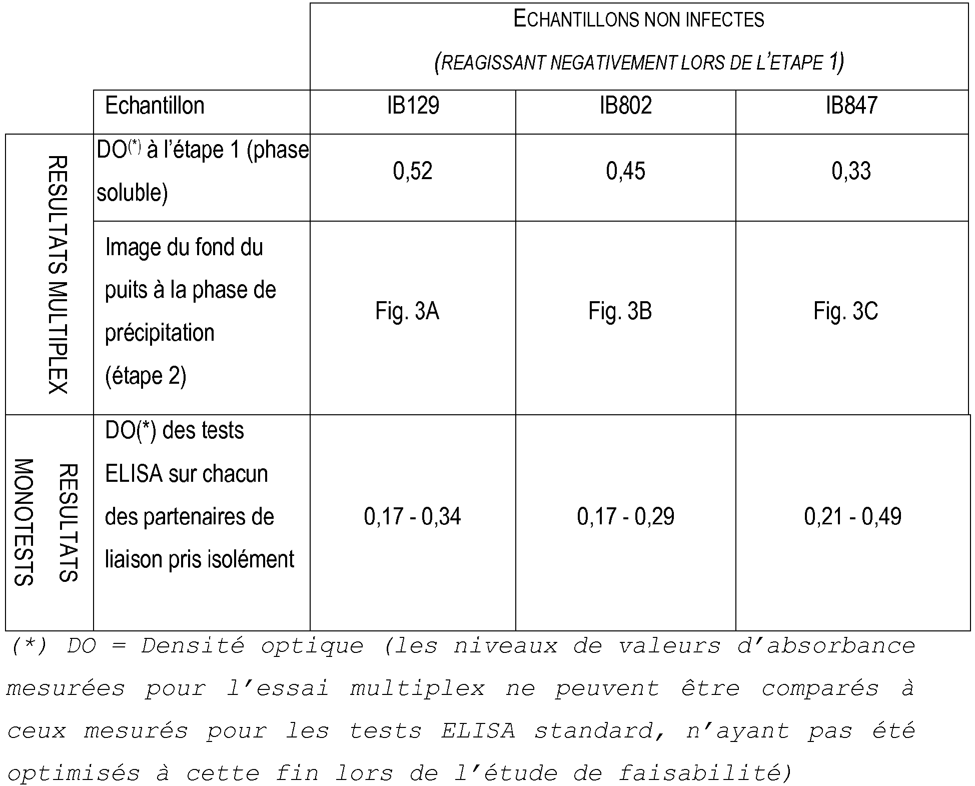

### II-3 Conclusions :

Aucun des spots de la matrice n'a réagi dans le cas des 3 derniers échantillons testés représentés ci-dessus (section II-2), ceux-ci étant tous séronégatifs pour les infections recherchées ici, comme cela est confirmé par les mesures d'absorbance des tests monoparamétriques réalisés sur l'ensemble des antigènes de la matrice.

Dans les trois premiers échantillons (section II-1), la présence de marqueurs infectieux a été révélée par l' « allumage » de certains spots, cohérent avec les résultats obtenus au niveau des tests monoparamétriques.

Les valeurs les plus basses de densité optique ont été observées au niveau des échantillons non infectieux, les valeurs supérieures à 0,6 étant associées à un statut infectieux caractérisé par les réactions visibles à des spots précis du puits.

Ces résultats expérimentaux confirment la potentialité de l'enzyme à catalyser successivement deux réactions chimiques par l'utilisation de deux substrats différents, révélant, sous deux formes différentes, la présence des mêmes complexes immunoenzymatiques formés au niveau de l'essai au cours des étapes a à c.

## Revendications

1. Procédé de détection et d'identification, dans un échantillon à l'état liquide, d'analyte(s) parmi au moins deux analytes, comprenant:
- dans une première étape, la réalisation d'un premier test immunoenzymatique donnant une réponse positive lorsqu'au moins un analyte est présent dans l'échantillon;
- la réalisation d'un second test immunoenzymatique mettant en oeuvre le complexe immunoenzymatique formé dans la première étape et permettant d'identifier le(s) analyte(s) dans l'échantillon,
et comprenant les étapes successives suivantes:
a) la mise en contact d'un échantillon avec un support coaté à l'aide des partenaires de liaison des au moins deux analytes recherchés;
b) l'élimination de l'échantillon;
c) l'ajout de(s) conjugué(s) enzymatique(s);
d) l'ajout d'un premier substrat de l'enzyme;
e) l'ajout d'un second substrat de l'enzyme,
dans lequel:
- dans le premier test immunoenzymatique, le premier substrat de l'enzyme est un substrat chromogène soluble;
- dans le second test immunoenzymatique, le second substrat de l'enzyme est un substrat chromogène précipitant;
- le second test immunoenzymatique, c'est a dire l'étape e), du procédé est mis en oeuvre de maniere systématique ou n'est réalisé que lorsque la réponse du premier test imunoenzymatique est positive.

2. Procédé de détection et d'identification selon l'une des revendications précédentes, dans lequel un des analytes est un antigène ou un anticorps.

3. Procédé de détection et d'identification selon l'une des revendications précédentes, dans lequel l'échantillon est du sang.

4. Procédé de détection et d'identification selon l'une des revendications précédentes, dans lequel le procédé est mis en oeuvre sur au moins deux échantillons en parallèle, l'un au moins des échantillons donnant avantageusement une réponse positive.

5. Procédé de détection et d'identification selon l'une des revendications précédentes, dans lequel un lavage est réalisé entre les deux tests immunoenzymatiques, avantageusement entre les étapes b), c), d) et e).

6. Procédé de détection et d'identification selon l'une des revendications précédentes, dans lequel les au moins deux analytes proviennent d'au moins deux agents infectieux différents.

7. Trousse pour la mise en oeuvre du procédé selon l'une des revendications 1 à 6 comprenant:
- un support sur lequel sont coatés les partenaires de liaison d'au moins deux analytes;
- une enzyme conjuguée à une ou plusieurs molécules aptes à interagir spécifiquement avec lesdits analytes;
- au moins deux substrats de ladite enzyme, dans lequel un soluble et un précipitant;
- éventuellement une solution de lavage.

8. Utilisation d'un procédé selon l'une des revendications 1 à 6 ou de la trousse selon la revendication 7 pour l'analyse de plusieurs sources d'analytes dans un échantillon ou pour l'analyse simultanée de plusieurs échantillons.

## Patentansprüche

1. Verfahren zum Erkennen und Identifizieren eines/von Analyten unter wenigstens zwei Analyten in einer Probe im flüssigen Zustand, das Folgendes beinhaltet:
- in einem ersten Schritt, Durchführung eines ersten immunenzymatischen Tests, der eine positive Antwort gibt, wenn wenigstens ein Analyt in der Probe vorhanden ist;
- Durchführung eines zweiten immunenzymatischen Tests, welcher den im ersten Schritt gebildeten enzymatischen Komplex verwendet und die Identifizierung des/der Analyten in der Probe erlaubt,
und das die folgenden aufeinander folgenden Schritte beinhaltet:
a) Inkontaktbringen einer Probe mit einem Träger, der mit Bindungspartnern von wenigstens zwei gesuchten Analyten beschichtet ist;
b) Eliminieren der Probe;
c) Zugeben eines/von enzymatischen Konjugats/-en;
d) Zugeben eines ersten Substrats des Enzyms;
e) Zugeben eines zweiten Substrats des Enzyms,
wobei:
- im ersten immunenzymatischen Test das erste Substrat des Enzyms ein lösliches chromogenes Substrat ist;
- im zweiten immunenzymatischen Test das zweite Substrat des Enzyms ein präzipitierendes chromogenes Substrat ist;
- der zweite immunenzymatische Test, das heißt Schritt e), des Verfahrens in systematischer Weise oder nur dann durchgeführt wird, wenn die Antwort des ersten immunenzymatischen Tests positiv ist.

2. Verfahren zum Erkennen und Identifizieren nach dem vorherigen Anspruch, bei dem einer der Analyten ein Antigen oder ein Antikörper ist.

3. Verfahren zum Erkennen und Identifizieren nach einem der vorherigen Ansprüche, bei dem die Probe Blut ist.

4. Verfahren zum Erkennen und Identifizieren nach einem der vorherigen Ansprüche, bei dem das Verfahren mit wenigstens zwei Proben parallel durchgeführt wird, wobei wenigstens eine der Proben vorteilhafterweise eine positive Antwort gibt.

5. Verfahren zum Erkennen und Identifizieren nach einem der vorherigen Ansprüche, bei dem ein Waschschritt zwischen zwei immunenzymatischen Tests vorteilhafterweise zwischen den Schritten b), c), d) und e) durchgeführt wird.

6. Verfahren zum Erkennen und Identifizieren nach einem der vorherigen Ansprüche, bei dem die wenigstens zwei Analyten von wenigstens zwei verschiedenen Infektionserregern stammen.

7. Kit zur Durchführung des Verfahrens nach einem der Ansprüche 1 bis 6, der Folgendes umfasst:
- einen Träger, der mit den Bindungspartnern von wenigstens zwei Analyten beschichtet ist;
- ein Enzym, konjugiert mit einem oder mehreren Molekülen, die spezifisch mit den genannten Analyten interagieren können;
- wenigstens zwei Substrate des genannten Enzyms, von denen eines löslich und ein anderes präzipitierend ist;
- gegebenenfalls eine Waschlösung.

8. Anwendung eines Verfahrens nach einem der Ansprüche 1 bis 6 oder Kit nach Anspruch 7 zum Analysieren von mehreren Analytquellen in einer Probe oder zum gleichzeitigen Analysieren mehrerer Proben.

## Claims

1. Method for detecting and identifying, in a sample in the liquid state, analyte(s) from among at least two analytes, comprising:
- in a first step, performing a first immunoenzymatic test which gives a positive response when at least one analyte is present in the sample;
- performing a second immunoenzymatic test which employs the immunoenzymatic complex formed in the first step and which allows the analyte(s) in the sample to be identified,
and comprising the following successive steps:
a) bringing a sample into contact with a support coated with the binding partners of the at least two analytes which are being sought;
b) removing the sample;
c) adding enzyme conjugate(s);
d) adding a first substrate of the enzyme;
e) adding a second substrate of the enzyme,
wherein:
- in the first immunoenzymatic test, the first substrate of the enzyme is a soluble chromogenic substrate;
- in the second immunoenzymatic test, the second substrate of the enzyme is a precipitating chromogenic substrate;
- the second immunoenzymatic test, that is to say step e), of the method is carried out systematically or is performed only when the response of the first immunoenzymatic test is positive.

2. Detection and identification method according to the preceding claim, wherein one of the analytes is an antigen or an antibody.

3. Detection and identification method according to any one of the preceding claims, wherein the sample is blood.

4. Detection and identification method according to any one of the preceding claims, wherein the method is carried out on at least two samples in parallel, at least one of the samples advantageously giving a positive response.

5. Detection and identification method according to any one of the preceding claims, wherein a washing is carried out between the two immunoenzymatic tests, advantageously between steps b), c), d) and e).

6. Detection and identification method according to any one of the preceding claims, wherein the at least two analytes come from at least two different infectious agents.

7. Kit for carrying out the method according to any one of claims 1 to 6, comprising:
- a support on which the binding partners of at least two analytes are coated;
- an enzyme conjugated to one or more molecules capable of interacting specifically with said analytes;
- at least two substrates of said enzyme, including a soluble substrate and a precipitating substrate;
- optionally a washing solution.

8. Use of a method according to any one of claims 1 to 6 or of the kit according to claim 7 for the analysis of a plurality of analyte sources in a sample or for the simultaneous analysis of a plurality of samples.
